# EUROPÄISCHE PATENTSCHRIFT

(11) **EP 3 288 469 B1**
(45) Veröffentlichungstag und Bekanntmachung des Hinweises auf die Patenterteilung: **08.11.2023**
(21) Anmeldenummer: 16719073.5
(22) Anmeldetag: 27.04.2016
(51) Int. Cl.: A61B 46/13, A61B 17/115, A61B 46/17, A61B 17/00

(54) **SCHUTZABDECKUNG FÜR EIN CHIRURGISCHES INSTRUMENT**
PROTECTIVE COVER FOR A SURGICAL INSTRUMENT
CAPUCHON DE PROTECTION POUR UN INSTRUMENT CHIRURGICAL

(30) Priorität: 28.04.2015 DE 202015003135 U; 28.04.2015 DE 202015003133 U
(43) Veröffentlichungstag der Anmeldung: 07.03.2018
(73) Patentinhaber: Ronchosan GmbH, 85653 Aying (DE)
(72) Erfinder: LANG, Reinhold, 82064 Straßlach (DE); MENGES, Christian, 82031 Grünwald (DE)
(74) Vertreter: Wallinger, Michael
(86) Internationale Anmeldenummer: PCT/EP2016/059405
(87) Internationale Veröffentlichungsnummer: WO 2016/174088

(56) Entgegenhaltungen:
- WO-A1-02/00121
- WO-A1-2011/063046

## Beschreibung

Die vorliegende Erfindung betrifft eine Schutzabdeckung für ein chirurgisches Instrument, insbesondere ein Klammernahtgerät, und eine Operationseinheit, bestehend aus einer solchen Schutzabdeckung und einem solchen chirurgischen Instrument, insbesondere einem Klammernahtgerät.

Eine Schutzabdeckung für chirurgische Instrumente und insbesondere für Klammernahtgeräte und Stapler ist mit der europäischen Patentschrift EP 2 049 037 B1 bekannt geworden. Diese Schutzabdeckung hat aber Nachteile und hat sich am Markt nicht durchsetzen können.

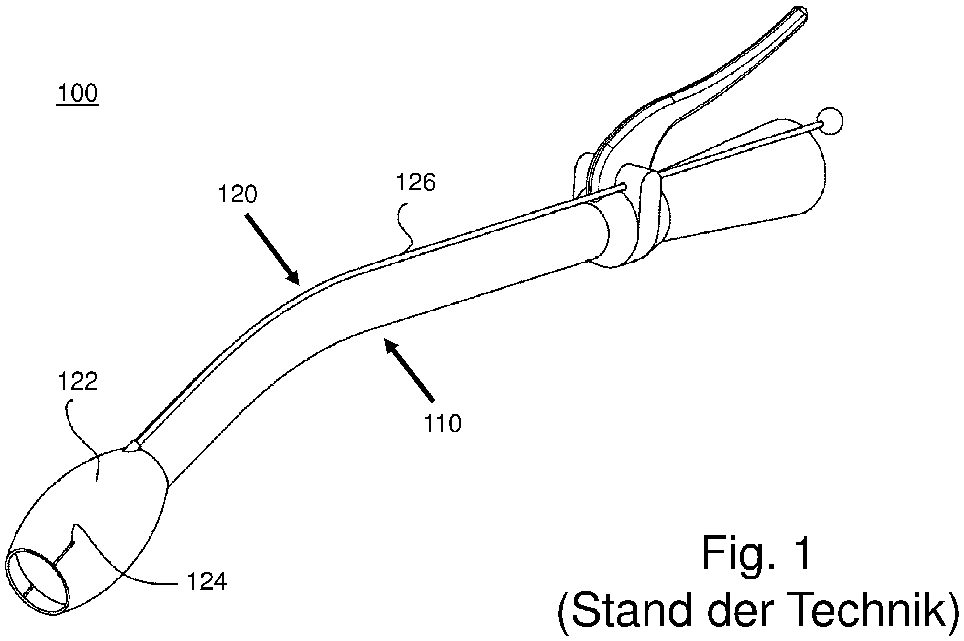

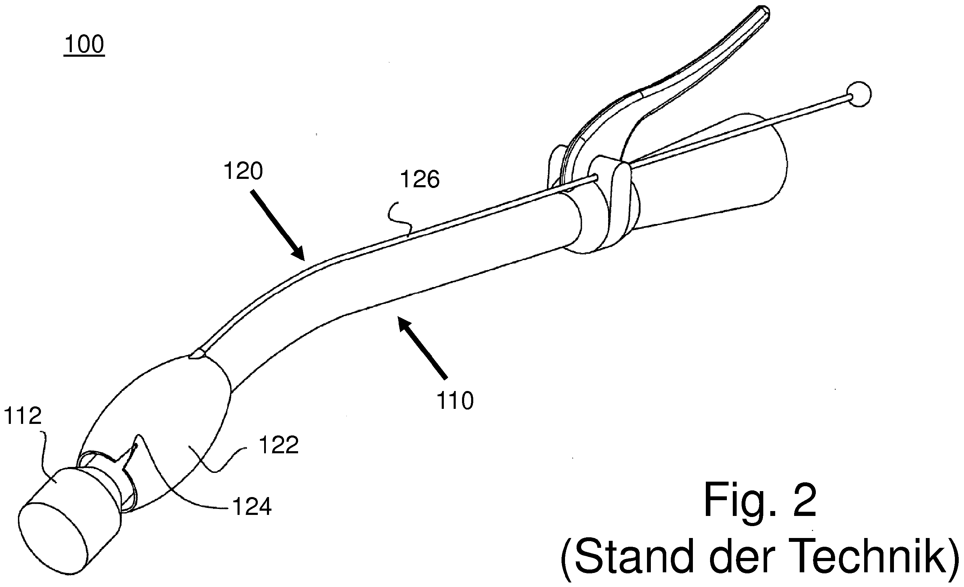

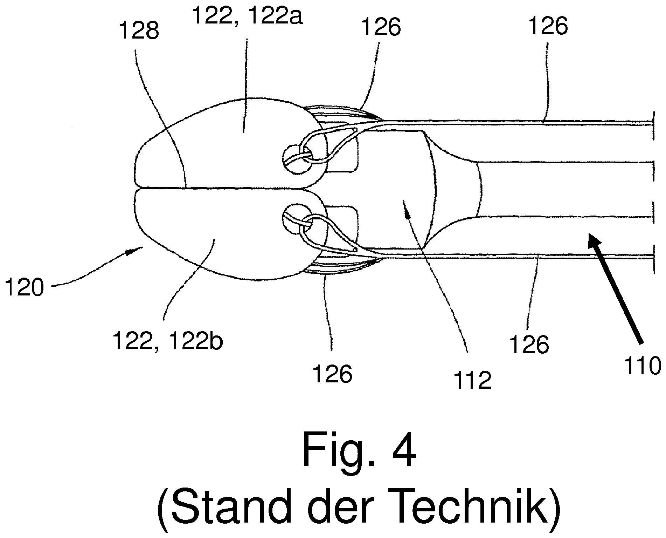

Die Funktionsweise dieser Schutzabdeckung 120 wird anhand der Fig. 1 und 2 der vorliegenden Anmeldung beschrieben, die den Figuren 2 und 4 der oben genannten Patentschrift entsprechen. Die olivenförmige Schutzabdeckung 120 weist eine Außenumhüllung 122 auf, die aus Metall oder Kunststoff bestehen kann. Diese Außenumhüllung 122 ist an ihrer Spitze mit einer Perforation 124 versehen. Die Schutzabdeckung 120 wird z.B. auf ein Klammernahtgerät aufgesetzt und mit geschlossener Perforation 124 in den Körper des Patienten an die entsprechende Operationsstelle vorgeschoben. Sobald das chirurgische Instrument 110 positioniert ist, wird die Perforation 124 über eine Zuganordnung 126 zum Reißen gebracht, wodurch sich im vorderen Teil der Außenumhüllung 122 eine Öffnung bildet bzw. vergrößert. Mittels der Zuganordnung 126 kann dann die Außenumhüllung 122 einstückig über den Kopfbereich 112 des chirurgischen Instruments 110 nach hinten gezogen werden, um den Kopfbereich 112 des chirurgischen Instrumentes 110 für den operativen Eingriff freizulegen.

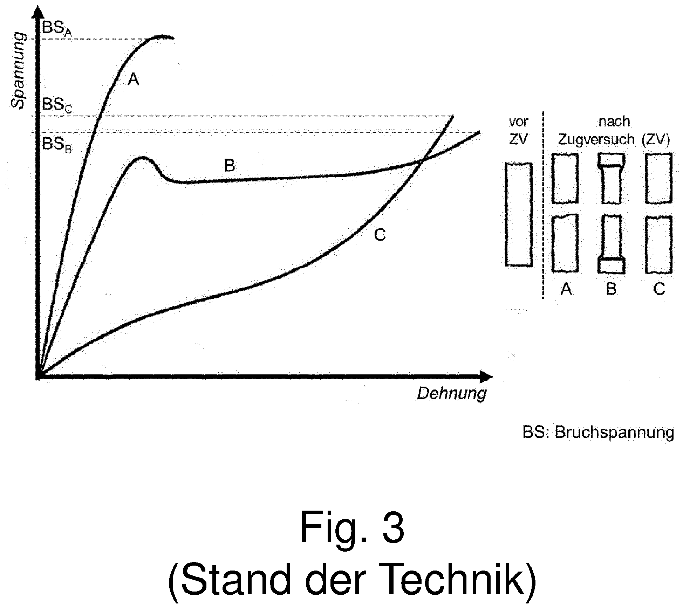

Die Fig. 3 zeigt ein typisches Kraft-Dehnungsdiagramm beim Zugversuch mit einem Kunststoff, entnommen aus: STOECKHERT, K. (HRSG.); WOEBCKEN, W. (HRSG.): Kunststoff-Lexikon. München: Carl Hanser Verlag, 9. Aufl., 1998, ISBN 3-446-17969-0.

Auf der Ordinate ist die Zugspannung aufgetragen, mit der ein aus Kunststoff bestehender Körper (rechts in Fig. 3) einer Zugbelastung unterworfen wird. Auf der Abszisse ist die Dehnung aufgetragen. Diese Kraft-Dehnungskurve ist von der Art des verwendeten Kunstoffs abhängig ist und kann für unterschiedliche Kunststoffe unterschiedliche Formen annehmen. Allen Kunststoffen, die für eine Schutzabdeckung der vorliegenden Art geeignet sind, wie z.B. Polyethylen, ist gemein, dass sich der Kunststoff dehnt bis es zum Bruch kommt.

Bei der Anwendung der Schutzabdeckung 120 mit dem chirurgischen Instrument 110 nach der EP 2 049 037 B1 bringt diese Charakteristik, die im Übrigen auch bei Metallen ähnlich verläuft, Probleme. Zum Aufreißen der Perforationen 124 hält der Chirurg das Klammernahtgerät (chirurgisches Instrument 110) z.B. mit der linken Hand und betätigt mit der rechten Hand die Zuganordnung 126. Dabei muss er eine immer größer werdende Kraft aufbringen, um die Perforation 124 entsprechend dem in Fig. 3 dargestellten Dehnungsverlauf zum Reißen zu bringen. Zu dem Zeitpunkt, an welchem die aufgebrachte Dehnung die sogenannte Bruchdehnung überschreitet, kommt es zum Aufbrechen der Perforationen, was sich dem Chirurgen vorher aufgrund der in Fig. 3 gezeigten Charakteristik nicht ankündigt. Dadurch sinkt die Gegenkraft der Zuganordnung 126 schlagartig auf Null ab. Damit gibt es für die mit der in diesem Beispiel linken Hand aufgebrachte Haltekraft keine Gegenkraft mehr und das chirurgische Instrument 110 wird nach den Gegebenheiten der Mechanik (*actio* = *reactio*) in Richtung auf den Patienten hin beschleunigt, d.h. wenn sich das Klammernahtgerät bereits plangemäß am Operationsort befindet, wird es dort zumindest kurzfristig einer Beschleunigung unterworfen. Da sich das chirurgische Instrument 110 dabei in einem durch Krankheit oder Verletzung bereits geschädigten Gewebebereich befindet, ist dieser Beschleunigungsstoß sehr unerwünscht.

Die Notwendigkeit, die Perforation 124 aufreißen zu müssen, schränkt auch die Auswahl des Materials, und zwar sowohl wenn Metall verwendet werden soll als auch insbesondere wenn Kunststoff verwendet werden soll, erheblich ein. Das Material muss so gewählt sein, dass das Aufreißen mit einer vom Chirurgen händisch aufzubringenden Kraft erfolgen kann.

Wird aber, insbesondere aus diesem Grund, ein eher elastisches Kunststoffmaterial gewählt und auch die Zuganordnung 126 aus diesem Material hergestellt, so entsteht beim Aufreißen der Perforation 124 zusätzlich ein Effekt, der in der Technik als "*Peitscheneffekt"* bekannt ist. Die Kunststoffbänder der Zuganordnung 126 sind - kurz bevor die Bruchdehnung der Perforation 124 erreicht ist - ebenfalls stark gedehnt. Da die Dehnkraft beim Bruch ebenfalls schlagartig auf Null zurückgeht, ziehen sich die Zugbänder entsprechend schlagartig zusammen, was zum Teil innerhalb des Körpers des Patienten passiert und deshalb ebenfalls hochproblematisch sein kann.

Ein weiterer Nachteil dieser Anordnung stellt die Herstellung der Perforation 124 dar. Sind die Aufreißkräfte der Perforation 124 zu gering, könnte die Perforation 124 schon beim Einführen der Schutzabdeckung 120 reißen, was zur Verletzung des Gewebes des Patienten führen kann. Sind die Perforationen 124 dagegen zu stark, lassen sie sich nicht aufreißen. Sie müssen deshalb sehr präzise durch Kunststoffspritzgießen oder durch einen nachfolgenden Vorgang des Bohrens oder Schneidens hergestellt werden. Der letztgenannte Vorgang ist zwar präzise, macht eine solche Schutzabdeckung 120 aber teuer.

Des Weiteren ist die olivenförmige Ausgestaltung der Schutzabdeckung 120 der EP 2 049 037 B1 in den meisten Anwendungsfällen von Nachteil, da bei gleichem Abdeckungsdurchmesser ein entsprechend größerer Körper vorgesehen werden muss, um einen ausreichenden Abdeckungsgrad zu erzielen. Dies gilt insbesondere auch deshalb, da die geometrischen Abmessungen chirurgischer Instrumente 110, insbesondere bei intraluminalen Operationen, ein besonders kritischer Parameter sind. Ein chirurgisches Instrument 110, welches möglicherweise generell geeignet wäre, bei einer konkreten Operation eingesetzt zu werden, wird durch die unnötig großen Abmessungen der Schutzabdeckung 120 im Verbund ungeeignet.

Eine andere Schutzabdeckung 120 für ein chirurgisches Instrument 110, insbesondere für ein Klammernahtgerät, ist mit der EP 1 294 291 B1 bekannt geworden. Diese Schutzabdeckung 120 wird in Bezug auf die Figuren 4 und 5 der vorliegenden Anmeldung beschrieben. Fig. 4 der vorliegenden Anmeldung entspricht der Fig. 8 der EP 1 294 291 B1 und zeigt den Kopfbereich des chirurgischen Instruments 110, das durch eine Schutzabdeckung 120 abgedeckt ist. Die Schutzabdeckung 120 besteht aus zwei Elementen 122a und 122b der Außenumhüllung 122, die durch eine Vielzahl von Sollbruchstellen 128 miteinander verbunden sind. Eine mit 126 bezeichnete Zuganordnung, die z.B. aus einer Schnur besteht, ist jeweils mit einem der Elemente 122a, 122b verbunden.

Auch hier positioniert der Chirurg die Operationseinheit 100, d.h. das chirurgische Instrument 110 mit der Schutzabdeckung 120, an der zu operierenden Stelle und betätigt dann die Zuganordnung 126. Wie in Fig. 5 der vorliegenden Anmeldung (Fig. 9 der EP 1 294 291 B1) gezeigt, reißen dadurch die Sollbruchstellen 128 und die Schutzabdeckung 120 teilt sich in wenigstens zwei Hälften (Elemente 122a, 122b) auf (Anspruch 1 der EP 1 294 291 B1), welche dann voneinander getrennt einzeln aus dem Körper auf einer undefinierten Bahn entfernt werden.

Somit besteht bei dieser Schutzabdeckung 120 das gleiche Problem wie bei der zuvor beschriebenen EP 2 049 037 B1. Der Chirurg muss auch hier mit der einen Hand eine Haltekraft und mit der anderen Hand eine Zugkraft aufbringen, um die Sollbruchstellen 128 zum Reißen zu bringen. Zusätzlich entsteht hier der Nachteil, dass die Schutzabdeckung 120 vollständig in zwei oder mehr Elemente 122a, 122b getrennt ist, die nicht mehr mit dem chirurgischen Instrument 110, d.h. dem Klammernahtgerät, verbunden sind. Dies kann für einige Operationen unproblematisch sein, für andere Operationen ist es zumindest aber problematisch, was eine universelle Anwendbarkeit der Schutzabdeckung 120 nach der EP 1 294 291 B1 ausschließt.

Ein Einführgerät für einen Stapler ist aus der WO 2011/063046 A1 bekannt.

Der vorliegenden Erfindung liegt die Aufgabe zugrunde, eine gegenüber dem zitierten Stand der Technik verbesserte Schutzabdeckung zur Verfügung zu stellen. Weiterhin ist es die Aufgabe der Erfindung, eine Operationseinheit bestehend aus einer solchen Schutzabdeckung und einem chirurgischen Instrument, insbesondere einem Klammernahtgerät, zur Verfügung zu stellen.

Diese Aufgabe wird erfindungsgemäß durch den Gegenstand des Anspruchs 1 gelöst. Eine erfindungsgemäße Operationseinheit ist Gegenstand des Anspruchs 12. Zu bevorzugende Weiterbildungen der Schutzabdeckung und der Operationseinheit sind Gegenstand der jeweiligen Unteransprüche.

Nach einem ersten Aspekt der vorliegenden Erfindung weist eine Schutzabdeckung für chirurgische Instrumente, insbesondere für Klammernahtgeräte, auf: eine Außenumhüllung, welche eingerichtet ist, wenigstens teilweise einen Kopfbereich des chirurgischen Instruments abzudecken; eine Betätigungseinrichtung, welche mit der Außenumhüllung wenigstens unidirektional kraftübertragend verbunden ist, und aus wenigstens einem Zugelement gebildet ist, wobei die Außenumhüllung aus wenigstens einem ersten Segment und aus wenigstens einem zweiten Segment gebildet ist, wobei das wenigstens eine erste Segment und das wenigstens eine zweite Segment mit Hilfe der Betätigungseinrichtung von einer Abdeck-Position in eine Freigabe-Position überführbar sind, wobei das wenigstens eine erste Segment und das wenigstens eine zweite Segment durch wenigstens ein Gelenk, insbesondere zwei Gelenke, miteinander verbunden sind.

Bei dem ersten Aspekt der vorliegenden Erfindung sind also zwei Varianten möglich: Bei der bevorzugten ersten Variante des ersten Aspekts hält das wenigstens eine Gelenk, insbesondere halten die zwei Gelenke, das wenigstens eine erste Segment und das wenigstens eine zweite Segment der Außenumhüllung in der Abdeckposition zusammen, ohne dass die Segmente durch einen Materialfilm, insbesondere einen Kunststoffilm oder einen Papierfilm oder einen Metallfilm oder einem Schichtverbund, oder Sollbruchstellen, insbesondere eine Perforation, zusammengehalten werden. Bei der zweiten Variante des ersten Aspekts wird die zusammenhaltende Wirkung des wenigstens einen Gelenks, insbesondere der zwei Gelenke, durch einen Materialfilm, insbesondere einen Kunststoffilm oder einen Papierfilm oder einen Metallfilm oder einem Schichtverbund, verbessert.

Nach einem zweiten Aspekt, für den kein Schutz beantragt wird, weist eine Schutzabdeckung für chirurgische Instrumente, insbesondere für Klammernahtgeräte, auf: eine Außenumhüllung, welche eingerichtet ist, wenigstens teilweise einen Kopfbereich des chirurgischen Instruments abzudecken; eine Betätigungseinrichtung, welche mit der Außenumhüllung wenigstens unidirektional kraftübertragend verbunden ist, und aus wenigstens einem Zugelement gebildet ist, wobei die Außenumhüllung aus wenigstens einem ersten Segment und aus wenigstens einem zweiten Segment gebildet ist, wobei das wenigstens eine erste Segment und das wenigstens eine zweite Segment mit Hilfe der Betätigungseinrichtung von einer Abdeck-Position in eine Freigabe-Position überführbar sind, wobei die Außenumhüllung einen Materialfilm aufweist, welcher in der Abdeckposition die beiden Segmente der Außenumhüllung miteinander verbindet.

Bei dem zweiten Aspekt der vorliegenden Erfindung sind ebenfalls zwei Varianten möglich: Bei der bevorzugten ersten Variante des zweiten Aspekts weist die Außenumhüllung einen Materialfilm auf, welcher in der Abdeckposition die beiden Segmente der Außenumhüllung miteinander verbindet, ohne dass die Segmente der Außenumhüllung zusätzlich durch wenigstens ein Gelenk, insbesondere zwei Gelenke, in der Abdeckposition zusammengehalten werden. Bei der zweiten Variante des zweiten Aspekts wird die zusammenhaltende Wirkung des Materialfilms durch wenigstens ein Gelenk, insbesondere zwei Gelenke, verbessert.

Die zweite Variante des ersten Aspekts entspricht somit in ihrer Ausgestaltung und Wirkungsweise der zweiten Variante des zweiten Aspekts.

Unter einem "chirurgischen Instrument" im Sinne der vorliegenden Erfindung werden insbesondere intraluminal eingesetzte Instrumente verstanden, wie sie beispielsweise in der minimalinvasiven Chirurgie verwendet werden. Sie weisen insbesondere einen Kopfbereich und einen Griffbereich auf, welcher insbesondere durch einen beliebig geformten, insbesondere ergonomisch geformten, rohr- oder stabförmigen Körper miteinander verbunden sind. Beispiele für chirurgische Instrumente im Sinne der vorliegenden Erfindung sind Kameras, Sonden, Spreizer, Klemmen, Schneidgeräte, insbesondere Messer, insbesondere Skalpelle, und Klammernahtgeräte.

Klammernahtgeräte im Sinne der vorliegenden Erfindung sind im Stand der Technik weithin bekannt, zum Beispiel in nachfolgenden Druckschriften: US2008169332A, US2010187283A, US2011036887A, US2013035683A, WO12177409A1, US2014058388A, US2005067459A, US2005067457A, US2005067458A, US2014350566A, US2005006429A, WO14033032A1, US2012323234A, US2005006431A, US2006049230A, DE102012103501A1, US2005184123A, US2009105735A, US2009204108A, US2004007608A, US2012325888A, US2005006430A, US2011152861A, US2005070958A, DE202011000617U1, DE202010000687U1, DE202010000688U1, WO11042508A1, DE202009018081U1, US2006011698A, US5865730A, US2005279804A, US2005184124A, US2002010482A, US2002188320A, US5814057A, DE10258925A1, US5040715A, DE10253771B3, DE10055923C2, US5129570A, DE19951940A1, US4955959A, DE29911266U1, US4978049A, US4805823A, US4633861A, US5221036A, US5452836A, DE4223162A1, DE424341 1 A1, DE9408749U1, US4714187A, US4605001A, US5015249A, US4776506A, DE9211975U1, DE9107166U1, US4608981A, US4930674A, DE3301713A1, DE3213304A1, DE8210178U1.

Unter einem "Kopfbereich" im Sinne der vorliegenden Erfindung wird insbesondere der Bereich eines chirurgischen Instruments verstanden, welcher das eigentliche Werkzeug, wie zum Beispiel das Messer, den Spreizer, die Klemme, die Kamera, die Klammervorrichtung etc. beinhaltet bzw. bildet.

Unter einer "Schutzabdeckung" im Sinne der vorliegenden Erfindung wird insbesondere eine Vorrichtung verstanden, welche sich zumindest in der Operationsphase des Ein- bzw. Hinführens an den Operationsort in einer Abdeck-Position befindet und dabei den Kopfbereich des chirurgischen Instruments wenigstens teilweise umgibt, um somit das umgebende Gewebe vor Beschädigungen durch Kanten chirurgischer Instrumente zu bewahren. Die Schutzabdeckung wird am Operationsort dann durch Betätigung durch den Chirurgen in eine Freigabe-Position überführt, in welcher der Kopfbereich des chirurgischen Instruments freiliegt und dieser so seiner originären Bestimmung entsprechend eingesetzt werden kann. In der Freigabeposition kann die Schutzabdeckung entweder vom chirurgischen Instrument losgelöst sein und separat davon entfernt werden oder während der Operation weiterhin mit diesem verbunden sein, um dann - in der Freigabeposition - gemeinsam entfernt zu werden oder die Schutzabdeckung kann derart ausgebildet sein, dass sie vor dem Entfernen reversibel zurück in die Abdeck-Position bewegt werden kann, um so das umgebende Gewebe auch während des Entfernens des chirurgischen Instruments vor möglichen Irritationen oder Verletzungen durch das chirurgische Instrument zu bewahren. Die Schutzabdeckung im Sinne der vorliegenden Erfindung weist eine Außenumhüllung und eine Betätigungseinrichtung auf.

Unter einer "Außenumhüllung" im Sinne der vorliegenden Erfindung wird insbesondere das Element der Schutzabdeckung verstanden, welches zumindest unmittelbar, insbesondere den Kopfbereich des chirurgischen Instruments, umgibt. Die Außenumhüllung ist eingerichtet, in der Abdeck-Position den Kopfbereich des chirurgischen Instruments zu umgeben und diesen in der Freigabe-Position freizugeben bzw. freizulegen. Die Außenumhüllung im Sinne der vorliegenden Erfindung ist aus wenigstens einem ersten Segment und aus wenigstens einem zweiten Segment gebildet.

Unter einem "Segment" im Sinne der vorliegenden Erfindung wird ein Element der Außenumhüllung verstanden, welches insbesondere sowohl in der Abdeckposition als auch in der Freigabeposition mit dem wenigstens einen anderen Segment verbunden bleibt. Nach einer Ausführung sind die beiden Segmente einteilig ausgebildet. Das wenigstens eine erste Segment und das wenigstens eine zweite Segment sind durch wenigstens ein Gelenk miteinander verbunden.

Unter einem "Gelenk" im Sinne der vorliegenden Erfindung wird ein Element der Außenumhüllung verstanden, welches das wenigstens eine erste Segment und das wenigstens eine zweite Segment der Außenumhüllung miteinander verbindet. Das Gelenk kann als separate Komponente oder einteilig bzw. integral zusammen mit dem wenigstens einen ersten Segment und dem wenigstens einen zweiten Segment ausgebildet sein. In letzterem Fall ist das Gelenk insbesondere in Form einer Verbindung zwischen den wenigstens zwei Segmenten realisiert, welche eingerichtet ist, während des gesamten Operationsverfahrens, d.h. insbesondere beginnend vom Einführen der chirurgischen Vorrichtung in den Körper bis zur Entfernung der chirurgischen Vorrichtung aus dem Körper, nicht zu brechen, sondern lediglich eine Relativbewegung der wenigstens zwei Segmente der Außenumhüllung zueinander zuzulassen, insbesondere eine Relativbewegung um lediglich eine Achse herum, welche im Wesentlichen senkrecht zur Vorschubrichtung des chirurgischen Instruments steht.

Unter einer "Vorschubrichtung" im Sinne der vorliegenden Erfindung wird diejenige Richtung verstanden, in der das chirurgische Instrument in den Körper vorgeschoben bzw. eingeschoben wird.

Unter einer "Betätigungseinrichtung" im Sinne der vorliegenden Erfindung wird ein System verstanden, welches an einem Ende mit der Außenumhüllung der Schutzabdeckung verbunden ist. Die Betätigungseinrichtung weist ein Kraftübertragungselement auf, welches eingerichtet ist, zumindest unidirektional direkt oder indirekt eine Kraft auf Veranlassung des Chirurgen bzw. eine durch den Chirurgen eingebrachte Kraft auf die Außenumhüllung zu übertragen, um die Außenumhüllung dazu zu veranlassen, sich von der Abdeck-Position in die Freigabe-Position zu bewegen. Insbesondere ist das Kraftübertragungselement ein Zugelement.

Unter einem "Zugelement" im Sinne der vorliegenden Erfindung wird ein Element verstanden, welches eingerichtet ist, zumindest eine Zugspannung zur Außenumhüllung hin zu übertragen. Das Zugelement kann hierbei ein- oder mehrteilig ausgebildet sein. Insbesondere ist wenigstens ein Teil des Zugelements zusammen mit wenigstens einem der Segmente der Außenumhüllung integral ausgebildet. Das Zugelement kann hierbei einen Stab, insbesondere einen Kunststoffstab, oder eine Schnur oder einen Draht, insbesondere einen Metalldraht, aufweisen, welcher an dem rohr- oder stabförmigen Körper des chirurgischen Instruments entlang und aus der Operationsöffnung hinaus geführt ist.

Unter einem "Materialfilm" im Sinne der vorliegenden Erfindung wird ein Materialabschnitt mit einer sehr geringen Abmessung hinsichtlich der Dicke, insbesondere dünner als 500 µm, insbesondere dünner als 100 µm, insbesondere dünner als 50 µm verstanden. Der Begriff "Materialfilm" umfasst dabei sowohl separat auf ein Trägerelement aufgebrachte Schichten desselben oder eines anderen Materials als dem Material des Trägerelements, wie hier: auf die Elemente der Außenumhüllung, als auch Materialausdünnungen des Trägerelements selbst, wie hier: zwischen den beiden Elementen der Außenumhüllung.

Die Schutzabdeckung für chirurgische Instrumente nach dem ersten Aspekt der vorliegenden Erfindung ist insbesondere vorteilhaft, da es somit ermöglicht wird, eine definierte Bewegung der Außenumhüllung, insbesondere der wenigstens zwei Segmente, zu ermöglichen. Gleichzeitig werden insbesondere die eingangs erwähnten stoßartigen Bewegungen des chirurgischen Instruments während der Überführung der Außenumhüllung von der Abdeck-Position in die Freigabe-Position zumindest vermindert. Die Handhabbarkeit der erfindungsgemäßen Operationseinheit, bestehend aus zumindest dem chirurgischen Instrument und der Schutzabdeckung, wird dadurch gegenüber der aus dem Stand der Technik bekannten Lösungen verbessert.

Die Schutzabdeckung für chirurgische Instrumente nach dem zweiten Aspekt, für den kein Schutz beantragt wird, ist insbesondere vorteilhaft, weil durch die Verwendung eines Materialfilms anstelle aus der EP 1 294 291 B1 vorbekannten Sollbruchstellen, insbesondere in Form von Steck- oder Stegbrücken, bei der Überführung der Schutzabdeckung von der Abdeck-Position in die Freigabe-Position keine, insbesondere keine scharfkantigen, Kanten an den Anlageflächen der Elemente der Schutzumhüllung entstehen, welche beim Entfernen der Schutzabdeckung aus dem Körper (unabhängig davon, ob das chirurgische Instrument gleichzeitig entfernt wird oder nicht) das umgebende Gewebe zumindest irritieren, insbesondere verletzen, insbesondere zerstören, können.

Erfindungsgemäß ist die Betätigungseinrichtung aus wenigstens einem ersten Zugelement und aus wenigstens einem zweiten Zugelement gebildet.

Dies ist insbesondere vorteilhaft, da somit die Krafteinleitung über die Betätigungseinrichtung in die Außenumhüllung, insbesondere in die wenigstens zwei Segmente, homogener erfolgt als bei einer einseitigen Krafteinleitung, sodass eine Seitbewegung des Kopfbereichs des chirurgischen Instruments innerhalb des menschlichen oder tierischen Körpers aufgrund der einseitigen Krafteinleitung vermieden wird. Nach einer bevorzugten Ausführung sind die wenigstens zwei Zugelemente gleichmäßig über den ellipsoiden, insbesondere kreisförmigen, Querschnitt, welchen die Außenumhüllung insbesondere an dem in Vorschubrichtung gesehen hinteren Bereich aufweist, verteilt.

Nach einer Ausführung der Schutzabdeckung, insbesondere mit dem Schutzfilm und ohne Materialausdünnung, in einem Anlagebereich liegen das wenigstens eine erste Segment und das wenigstens eine zweite Segment in dem Anlagebereich aneinander, insbesondere lose, an.

Dies ist insbesondere vorteilhaft, da somit stoßartige Bewegungen des chirurgischen Instruments während der Überführung der Außenumhüllung von der Abdeck-Position in die Freigabe-Position durch den Wegfall eines Reißens oder eines Trennens einer formschlüssigen Verbindung, insbesondere einer Steckverbindung, zumindest vermindert, insbesondere weiter vermindert, werden. Aufgrund des Wegfalls der Sollbruchstellen, wie sie beispielsweise in der EP 1 294 291 B1 gezeigt sind, wird auch zusätzlich das Verletzungsrisiko beim Entfernen der Schutzabdeckung vermindert, da die wenigstens zwei Segmente in ihrem Anlagebereich auch nach der Überführung in die Freigabe-Position weiterhin glatte Kanten aufweisen, welche nicht durch teilweise scharfkantige Fragmente ehemaliger Sollbruchstellen unterbrochen sind.

Nach einer Ausführung weist die Betätigungseinrichtung wenigstens einen ersten Vorsprungsabschnitt auf, welcher an dem wenigstens einen ersten Zugelement angeordnet ist, und wenigstens einen zweiten Vorsprungsabschnitt, welcher an dem wenigstens einen zweiten Zugelement angeordnet ist.

Unter einem "Vorsprungsabschnitt" im Sinne der vorliegenden Erfindung wird im geometrischen Sinne ein Vorsprung verstanden, welcher auch der Versteifung dienen kann. Der Vorsprungsabschnitt ist insbesondere derart gestaltet, dass er mit einer Oberfläche an dem chirurgischen Instrument anliegt. Der Vorsprungsabschnitt ist insbesondere in Vorschubrichtung gesehen hinter dem wenigstens einen Gelenk angeordnet und entweder direkt oder indirekt mit wenigstens einem der Elemente verbunden.

Dies ist insbesondere vorteilhaft, da der Vorsprungsabschnitt, welcher in Vorschubrichtung gesehen hinter dem wenigstens einen Gelenk angeordnet ist und welcher entweder direkt oder indirekt mit wenigstens einem der Elemente verbunden ist, eine Relativbewegung der wenigstens zwei Elemente zueinander verhindert. Dies wird durch folgenden technischen Effekt begründet: Würde eine ungewollte Kraft die wenigstens zwei Elemente der Außenumhüllung aus der Abdeck-Position in Richtung der Freigabe-Position bewegen wollen, wird über das wenigstens eine Gelenk ein Drehmoment auf das wenigstens eine Zugelement übertragen. Ausgehend von einer Öffnungsbewegung der wenigstens zwei Elemente, welche durch das wenigstens eine Gelenk umgelenkt wird, ergibt sich eine Bewegung des wenigstens einen Zugelements in Richtung auf das chirurgische Instrument zu. Diese Bewegung des wenigstens einen Zugelements wird durch den Vorsprungsabschnitt verhindert, da dieser durch seine Anlage am chirurgischen Instrument ein Gegenmoment erzeugt, welcher dem durch die ungewollte Kraft induzierten Moment entgegenwirkt.

Nach einer Ausführung ist der Vorsprungsabschnitt derart überdimensioniert gestaltet, dass er nicht nur den ohne Vorsprungsabschnitt bestehenden Abstand zwischen Zugelement und chirurgischem Instrument überbrückt, sondern einen Anpressdruck gegenüber dem chirurgischen Instrument erzeugt, welcher das Zugelement im Bereich des Vorsprungselements von dem chirurgischen Instrument wegdrückt. Durch dieses Wegdrücken wird über das Zugelement und das wenigstens eine Gelenk ein Biegemoment auf wenigstens eines der Elemente übertragen, welches die wenigstens zwei Elemente der Außenumhüllung aufeinander zu drückt und somit ein ungewolltes Auseinanderklaffen der wenigstens zwei Elemente der Außenumhüllung verhindert.

Erfindungsgemäß ist das wenigstens eine erste Segment mit dem wenigstens einen ersten Zugelement über einen ersten Vorsprungsabschnitt verbunden und das wenigstens eine zweite Segment mit dem wenigstens einen zweiten Zugelement über einen zweiten Vorsprungsabschnitt verbunden.

Dies ist insbesondere vorteilhaft, um die Vorteile der oben bereits beschriebenen gleichmäßigen Krafteinleitung mit den Vorteilen des oben bereits beschriebenen "Auseinanderklaff-Schutzes" miteinander zu verbinden.

Nach einer Ausführung sind der wenigstens eine erste Vorsprungsabschnitt und der wenigstens eine zweite Vorsprungsabschnitt eingerichtet, das wenigstens eine erste Segment und das wenigstens eine zweite Segment in ihrer Position zu halten, bis eine Kraft über die Betätigungseinrichtung an das Segment, insbesondere die Segmente, übertragen wird.

Dies ist insbesondere vorteilhaft, da der Vorsprungsabschnitt bewirkt, dass sich das wenigstens eine Segment nur dann um die oben beschriebene Gelenkachse herum drehen kann, wenn sich das wenigstens eine Zugelement bewegt, insbesondere in einer Richtung im Wesentlichen parallel zur Vorschubrichtung des chirurgischen Instruments bewegt. Mit anderen Worten: Nach einer Ausführung ist das Zugelement ausgerichtet, eine Kraft bidirektional zu übertragen. Der Vorsprungsabschnitt ist eingerichtet, ein Auseinanderklaffen wenigstens eines Segments relativ zu dem wenigstens einen anderen Segment zu verhindern. Somit wird eine Relativbewegung des wenigstens einen Segments um die durch das Gelenk definierte Gelenkachse bzw. die durch das Gelenk definierten Gelenkachsen herum verhindert, wenn das dem wenigstens einem Segment entgegengesetzte Ende des Zugelements in beliebiger Weise arretiert und/oder fixiert ist.

Nach einer Ausführung ist der wenigstens eine erste Vorsprungsabschnitt und der wenigstens eine zweite Vorsprungsabschnitt durch eine Querschnittsverdickung der Betätigungseinrichtung, insbesondere einteilig, gebildet.

Dies ist insbesondere vorteilhaft, da somit auf die Herstellung und Montage zusätzlicher Komponenten an der Schutzabdeckung vermieden werden können. Dies reduziert die Produktionskosten und erhöht die Komplexität des Herstellungsverfahrens der Schutzabdeckung nur geringfügig, insbesondere im Wesentlichen nicht.

Nach einer Ausführung weisen das wenigstens eine erste Segment und das wenigstens eine zweite Segment in Vorschubrichtung gesehen hinter dem wenigstens einen Gelenk jeweils eine Öffnungsbegrenzung auf, welche die Entfernung der in Vorschubrichtung gesehen vorderen Bereiche der Segmente zueinander in der Freigabeposition begrenzt.

Dies ist insbesondere vorteilhaft, da somit ein Verklemmen der Schutzabdeckung mit dem chirurgischen Instrument vermieden werden kann. Durch die Begrenzung des Öffnungswinkels wird die Relativbewegung der wenigstens zwei Segmente während ihrer Überführung von der Abdeck-Position in die Freigabe-Position nicht erst durch einen Teil, insbesondere den oben erwähnten rohr- oder stabförmigen Körper, sondern bereits vorher gestoppt. Somit besteht auch in der Freigabe-Position ein Abstand zwischen dem chirurgischen Instrument und den Elementen, welcher es ermöglicht, die Schutzabdeckung - sofern gewünscht - aus dem Körper herauszuziehen ohne dass eine nennenswerte Reibkraft zwischen Schutzabdeckung und chirurgischem Instrument überwunden werden muss.

Nach einer Ausführung besteht die Schutzabdeckung im Wesentlichen, insbesondere vollständig, aus Kunststoff.

Dies ist insbesondere vorteilhaft, da Kunststoffteile vergleichsweise günstig mittels Spritzgießverfahren hergestellt werden können. Dies ist auch in der Fähigkeit dieser Technologie begründet, urformend direkt eine zumindest im Wesentlichen nacharbeitungsfreie Endgeometrie bereitzustellen. Des Weiteren haben ausgewählte Kunststoffe, insbesondere thermoplastische Kunststoffe, insbesondere Kunststoffe, welche wenigstens Spuren von Propylen aufweisen, insbesondere Kunststoffe, welche im Wesentlichen aus Polypropylen bestehen, insbesondere Polypropylen, insbesondere die Eigenschaft sterilisierbar zu sein und weisen insbesondere die erforderlichen Zulassungen für den Einsatz im medizinischen Bereich auf.

Nach einer Ausführung ist die Außenumhüllung eingerichtet, das chirurgische Instrument in seinem Kopfbereich, insbesondere in der Vorschubrichtung von vorne gesehen, im Wesentlichen vollständig, insbesondere vollständig, abzudecken.

Dies ist insbesondere vorteilhaft, da gerade der Kopfbereich eines chirurgischen Instruments aufgrund seiner mitunter vielfältigen Funktionen in seiner Geometrie sehr komplex ist. Deshalb kann bei der Entwicklung medizinischer Instrumente nicht immer im gewünschten Maße auf die Ergonomie Rücksicht genommen werden, da die funktionalen Aspekte im Vordergrund stehen (müssen). Hier setzt die Außenumhüllung an, welche die fehlende Ergonomie zumindest während der Hinführung des chirurgischen Instruments zum Operationsort bereitstellt. Je besser, d.h. je vollständiger, der Kopfbereich des chirurgischen Instruments von der Außenumhüllung umgeben ist, desto schonender kann das chirurgische Instrument zum Operationsort gebracht werden.

Nach einer Ausführung weisen die Zugelemente ein Halteelement auf, welches eingerichtet ist, die Zugelemente an dem chirurgischen Instrument zu fixieren, insbesondere in axialer Richtung verschiebbar zu fixieren.

Dies ist insbesondere vorteilhaft, da die Zugeelemente auf diese Weise während der Verbindung mit dem chirurgischen Instrument, insbesondere während der Überführung der Außenumhüllung von der Abdeck-Position in die Freigabe-Position geführt sind. Auch während des Ein- und Ausführens liegen die Zugelemente auf diese Weise bevorzugt eng am chirurgischen Element an, wodurch ein Verheddern oder Verschlingen miteinander oder mit umgebendem Gewebe verhindert wird.

Nach einer Ausführung verbindet das Halteelement die Zugelemente in einer Richtung quer zur Vorschubrichtung des chirurgischen Instruments miteinander.

Dies ist insbesondere vorteilhaft, da das eine Halteelement für die Mehrzahl an Zugelementen, insbesondere für beide Zugelemente, gemeinsam eine Fixierungswirkung der oben beschriebenen Art bereitstellt.

Nach einer Ausführung ist das Haltelement dazu eingerichtet die Zugelemente so miteinander zu verbinden, dass über das Halteelement Kräfte zwischen der Mehrzahl an Zugelementen übertragen werden kann.

Dies ist insbesondere vorteilhaft, da somit eine gleichmäßige Krafteinwirkung über die Zugelemente auf die Segmente der Außenumhüllung begünstigt wird, was zu einer Vereinfachung der Handhabung der Schutzabdeckung führt.

Nach einer Ausführung weist die Schutzabdeckung in ihrer geometrischen Gestaltung eines in Vorschubrichtung gesehen vorderen Bereichs ein Kugelsegment auf, welches insbesondere im Wesentlichen halbkugelförmig ausgebildet ist, insbesondere halbkugelförmig ausgebildet ist.

Dies ist insbesondere vorteilhaft, da diese Geometrieformen gute intraluminale Gleiteigenschaften mit einer guten Raumausnutzung kombinieren. Somit wird auf der einen Seite das Irritiations- und Verletzungsrisiko für das umgebende Gewebe insbesondere während der Vorschubbewegung verringert und auf der anderen Seite werden die geometrischen Abmessungen der Operationseinheit nicht unnötig vergrößert.

Nach einer Ausführung ist der Materialfilm als Schutzfilm und/oder als Materialausdünnung ausgebildet.

Dies ist insbesondere vorteilhaft da somit der Materialfilm an die jeweiligen spezifischen Gegebenheiten der anstehenden Operationsaufgabe angepasst werden kann. Eine Materialausdünnung ist insbesondere vorteilhaft, da somit zusätzliche Arbeitsschritte während der Herstellung der Schutzabdeckung vermieden werden können, was zu einer wirtschaftlich rentablen Herstellbarkeit der Schutzabdeckung beiträgt. Ein Schutzfilm ist insbesondere vorteilhaft, da auf diese Weise ein anderes Material eingesetzt werden kann als das Material der Elemente der Außenumhüllung, wodurch weitere Eigenschaften, wie zum Beispiel verbesserte Gleiteigenschaften, Abdichtungseigenschaften oder optische Eigenschaften, wie zum Beispiel Farbe, realisiert werden können.

Nach einer Ausführung ist die Materialausdünnung integral mit dem wenigstens einen ersten Segment der Außenumhüllung und dem wenigstens einen zweiten Segment der Außenumhüllung gebildet.

Nach einer Ausführung ist die Materialausdünnung dergestalt ausgebildet, dass die Materialausdünnung mit den umgebenden Elementen der Außenumhüllung in Vorschubrichtung von vorne gesehen eine ebene Oberfläche bildet.

Dies ist insbesondere vorteilhaft, da somit die Gleiteigenschaften der Schutzabdeckung weiter verbessert werden.

Nach einer Ausführung erstreckt/erstrecken sich die Materialausdünnung und/oder der Schutzfilm nicht über den gesamten Anlagebereich der umgebenden Elemente der Außenumhüllung.

Nach einer Ausführung ist der Materialfilm aus einem Kunststoffilm oder einem Papierfilm oder einem Metallfilm oder einem Schichtverbund der vorgenannten Filmarten gebildet.

Dies ist insbesondere vorteilhaft, da somit die Schutzabdeckung an die spezifischen Anforderungen der anstehenden Operationsaufgabe optimal angepasst werden kann, da die oben genannten Alternativen oder Kombinationen derselben unabhängig vom Material der Schutzabdeckung, insbesondere der Elemente der Außenumhüllung, gewählt werden können, um somit die für die anstehende Operationsaufgabe erforderlichen Eigenschaften in optimaler Weise bereitstellen zu können.

Nach einer Ausführung ist der Schutzfilm an der Außenumhüllung befestigt, insbesondere aufgeklebt.

Dies ist insbesondere vorteilhaft, da somit der Schutzfilm an der Außenumhüllung in einfacher und kostengünstiger Weise in seiner Position gehalten werden kann. Als Klebstoffe kommen all diejenigen Klebstoffe in Betracht, welche die erforderliche Verbindungsfestigkeit bereitstellen können, und insbesondere welche eine Zulassung zur Verwendung für medizinische Produkte aufweisen.

Nach einer Ausführung ist der Schutzfilm auf die Außenumhüllung aufgetragen, insbesondere aufgesprüht oder mittels Dampfabscheidung aufgetragen.

Dies ist insbesondere vorteilhaft, da somit die Dicke des Schutzfilms besonders dünn eingestellt werden kann, was die Reißeigenschaften während der Überführung von der Abdeck-Position in die Freigabe-Position erheblich verbessert, d.h. insbesondere die erforderliche aufzubringende Kraft, um ein Reißen zu bewirken, reduziert. Des Weiteren ist das Dampfabscheiden von Vorteil, da somit auf Klebstoffe verzichtet werden kann und der Film in optimaler Weise der Geometrie der Außenumhüllung folgt.

Nach einer Ausführung ist der Schutzfilm in der Vorschubrichtung von vorne gesehen in dem von der Außenumhüllung wenigstens teilweise abgedeckten Kopfbereich des chirurgischen Instruments durchgängig, d.h. ohne Durchbrechungen, insbesondere ohne Perforationen.

Dies ist insbesondere vorteilhaft, da es somit ermöglicht wird, den mitunter komplexen und hinsichtlich Verschmutzung empfindlichen Kopfbereich des chirurgischen Instruments in dem von der Außenumhüllung wenigstens teilweise abgedeckten Kopfbereich des chirurgischen Instruments vor dem Eindringen von Schmutz, z.B. Darminhalt oder dergleichen, zu schützen.

Nach einer Ausführung weist der Schutzfilm keine Perforationen auf.

Dies ist insbesondere vorteilhaft, da somit der Schutz des mitunter komplexen und hinsichtlich Verschmutzung empfindlichen Kopfbereichs des chirurgischen Instruments in dem von der Außenumhüllung wenigstens teilweise abgedeckten Kopfbereich des chirurgischen Instruments vor dem Eindringen von Schmutz, z.B. Darminhalt oder dergleichen, geschützt ist, insbesondere noch besser geschützt ist.

Nach einer Ausführung weist die Schutzabdeckung zwei Gelenke auf, welche in Vorschubrichtung gesehen in einem hinteren Bereich der Außenumhüllung, und insbesondere an zwei einander in radialer Richtung gesehen entgegengesetzten Positionen, angeordnet sind.

Dies ist insbesondere vorteilhaft, da somit die Geometrie der Gelenke stark vereinfacht werden kann. Durch die Positionierung der Gelenke ist es - sofern dies gewünscht ist - nicht erforderlich, dass ein Gelenk allein eine Verschwenkachse der wenigstens zwei Segmente der Außenumhüllung zueinander realisiert. Durch die gelehrte besondere Anordnung können auch punktförmige Gelenke oder Kugelgelenke eingesetzt werden, da sich die von ihnen einzeln nicht eingeschränkten Freiheitsgrade im Verbund auf die eine gewünschte Verschwenkachse reduzieren. Dies vereinfacht die Auslegung und Herstellung der Schutzabdeckung und trägt so zu einer wirtschaftlich rentablen Herstellbarkeit bei.

In einer bevorzugten Ausführung weist die Schutzabdeckung genau zwei Segmente auf, die über genau zwei Gelenke, welche an zwei einander in radialer Richtung entgegengesetzten Positionen, angeordnet sind, auf. Dies ist vorteilhaft, da auch in der Freigabe-Position durch die beiden Segmente und die beiden Gelenke ein durchgängiger Ring um das chirurgische Instrument herum gebildet ist, welcher ein geführtes Herausziehen der Schutzabdeckung - sofern gewünscht - ermöglicht.

Nach einer Ausführung deckt der Schutzfilm den Anlagebereich des wenigstens einen ersten Abschnitts und des wenigstens einen zweiten Abschnitts in der Vorschubrichtung von vorne gesehen vollständig ab, insbesondere dichtet er diesen wasserdicht ab, insbesondere dichtet er diesen luftdicht ab.

Dies ist insbesondere vorteilhaft, da es somit ermöglicht wird, den mitunter komplexen und hinsichtlich Verschmutzung empfindlichen Kopfbereich des chirurgischen Instruments vor dem Eindringen von Schmutz, z.B. Darminhalt oder dergleichen, Flüssigkeit oder gegebenenfalls Gasen zu schützen. Hierbei gilt zu beachten, dass hierdurch lediglich ein Abdichten in Vorschubrichtung von vorne gesehen gemeint ist. Eine vollständige Abdichtung des Kopfbereichs des chirurgischen Instruments gegen ein Eindringen von Schmutz, Flüssigkeit oder Gas auch von in Vorschubrichtung von hinten gesehen ist zwar möglich, aber nicht vom obigen Wortlaut gefordert.

Eine Operationseinheit gemäß der vorliegenden Erfindung weist auf: ein chirurgisches Instrument, insbesondere ein Klammernahtgerät; und eine Schutzabdeckung, wobei die Schutzabdeckung an dem chirurgischen Instrument angeordnet ist und dazu beiträgt, die Gefahr der Verletzung von menschlichem oder tierischem Gewebe bei einer Vorschubbewegung des chirurgischen Instruments im menschlichen oder tierischen Körper zu vermindern.

Unter einer "Operationseinheit" im Sinne dieser Erfindung ist ein Verbund aus chirurgischem Instrument und Schutzabdeckung zu verstehen. Für die Vorzüge und Definitionen der einzelnen Merkmale der erfindungsgemäßen Operationseinheit sei auf die obigen Ausführungen verwiesen, welche hier in gleichem Maße gelten.

Nach einer Ausführung besteht die Schutzabdeckung wenigstens im Wesentlichen aus Kunststoff, insbesondere vollständig aus Kunststoff, und/oder die Schutzabdeckung ist so gestaltet, dass sie zumindest einen Kopfbereich des chirurgischen Instruments wenigstens teilweise abdeckt.

Nach einer Ausführung sind wenigstens zwei getrennt sterilisierte Verpackungseinheiten, nämlich wenigstens ein chirurgisches Instrument, insbesondere ein Klammernahtgerät, und wenigstens eine Schutzabdeckung vorgesehen.

Dies ist insbesondere vorteilhaft, da verschiedene Ausführungsformen von chirurgischen Instrumenten mit verschiedenen Ausführungsformen an Schutzabdeckungen je nach dem spezifischen Operationsziel miteinander kombiniert werden können. Eine integrale Lösung, wie sie beispielsweise die EP 2 049 037 B1 lehrt, führt dazu, dass das Einsatzgebiet des mitunter sehr teuren chirurgischen Instruments stark eingeschränkt ist, sodass eine Vielzahl solcher verschiedener integrierter Operationseinheiten mit dem entsprechenden finanziellen Aufwand bereitgehalten werden müssen, um ein vergleichbares Operationsspektrum abdecken zu können.

Nach einer Ausführung ist die Schutzabdeckung eine Schutzabdeckung entsprechend der vorstehenden Ausführungen.

Zu den Vorteilen dieser Ausgestaltungen einer Schutzabdeckung wird auf die entsprechenden vorstehenden Ausführungen verwiesen.

Nach einer Ausführung ist die Operationseinheit zur Verwendung bei wenigstens einer der folgenden Indikationen, insbesondere Klammernahtverbindungen, vorgesehen:
(a) Speiseröhre zu Magen und Magen zu Speiseröhre;
(b) Enddarm zu Dickdarm und Dickdarm zu Enddarm;
(c) Dickdarm zu Dickdarm;
(d) Dickdarm zu Dünndarm und Dünndarm zu Dickdarm;
(e) Dünndarm zu Dünndarm;
(f) Magen zu Dünndarm und Dünndarm zu Magen;
(g) Proktologische Eingriffe, insbesondere zur Schleimhautabtragung; und
(h) Vollwandresektionen.

Dies ist insbesondere vorteilhaft, da die Operationseinheit nach dieser Ausführung für viele verschiedene Operationsarten eingesetzt werden kann, sodass sich der Chirurg nicht ständig an die Handhabung neuer chirurgischer Instrumente gewöhnen muss und auch die Krankenhäuser die Anzahl der vorzuhaltenden Formen verschiedener nicht-erfindungsgemäßer Operationseinheiten reduziert werden kann.

Weitere vorteilhafte Weiterbildungen der vorliegenden Erfindung ergeben sich aus den Unteransprüchen und der nachfolgenden Beschreibung bevorzugter Ausführungen. Hierzu zeigt, teilweise schematisiert:
- Fig. 1: eine Operationseinheit aus dem Stand der Technik gemäß EP 2 049 037 B1 mit einer Außenumhüllung in einer Abdeck-Position;
- Fig. 2: die Operationseinheit der Fig. 1 mit der Außenumhüllung in einer Freigabe-Position;
- Fig. 3: Kraft-Dehnungsdiagramm aus dem Stand der Technik bei einem Zugversuch mit einem Kunststoff;
- Fig. 4: eine Operationseinheit aus dem Stand der Technik gemäß EP 1 294 291 B1 mit einer Außenumhüllung in einer Abdeck-Position;

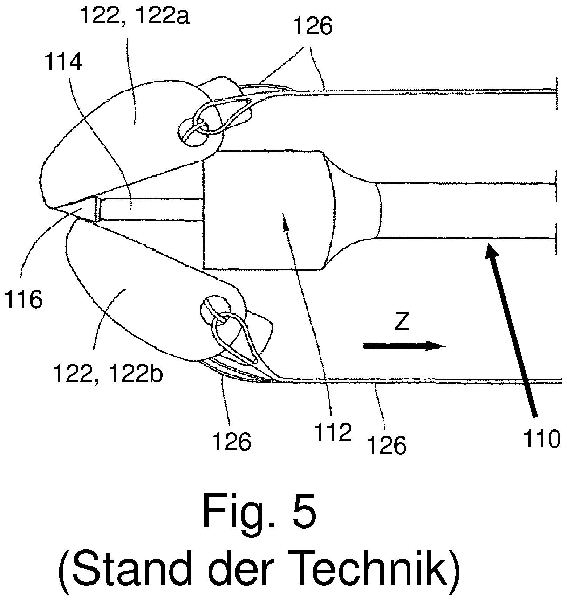

- Fig. 5: die Operationseinheit der Fig. 4 mit der Außenumhüllung in einer Freigabe-Position;
- Fig. 6: eine Schutzabdeckung nach einer ersten Ausführungsform der vorliegenden Erfindung in einer Seitenansicht;
- Fig. 7: die Schutzabdeckung der Fig. 6 in einer Detailansicht;
- Fig. 8: eine Schutzabdeckung nach einer zweiten Ausführungsform der vorliegenden Erfindung in einer Detailansicht;
- Fig. 9: eine Schutzabdeckung nach einer dritten Ausführungsform der vorliegenden Erfindung in einer Detailansicht;
- Fig. 10: eine Schutzabdeckung nach einer vierten Ausführungsform, nicht Teil der Erfindung, in einer Seitenansicht (rechte Darstellung) und in einer Vorderansicht aus der Vorschubrichtung (linke Darstellung), jeweils in der Abdeck-Position;
- Fig. 11: die Schutzabdeckung der Fig. 10 in der Freigabe-Position;
- Fig. 12: eine Detailansicht des Anlagebereichs der Fig. 10; und
- Fig. 13: eine Schutzabdeckung nach einer fünften Ausführungsform, nicht Teil der Erfindung, in einer Seitenansicht.

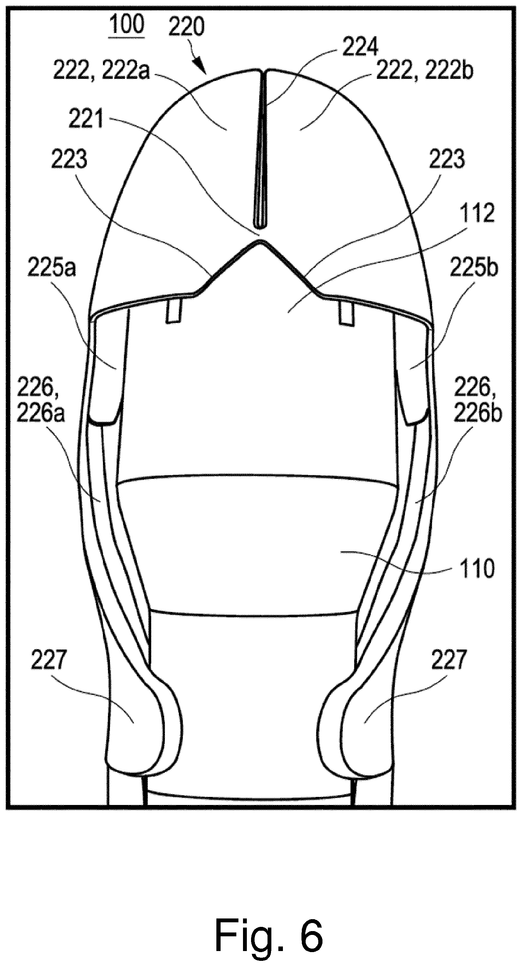

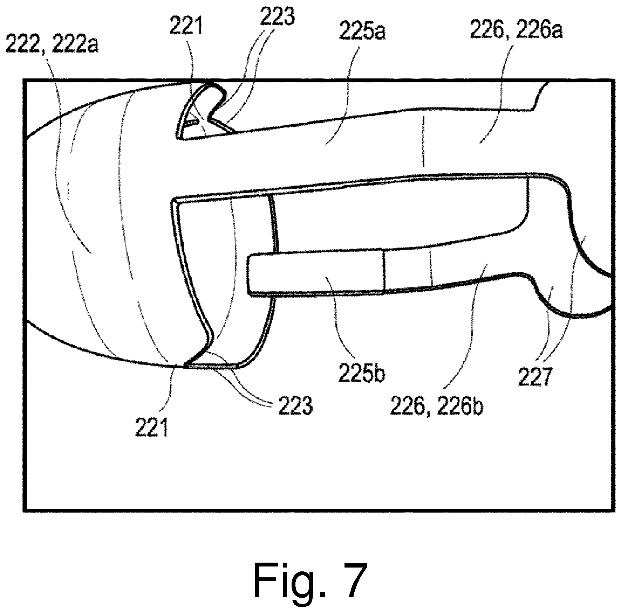

Fig. 6 und 7 zeigen eine Schutzabdeckung 220 nach einer ersten Ausführungsform der vorliegenden Erfindung in zwei verschiedenen Seitenansichten jeweils in der Abdeckposition. Die Schutzabdeckung 220 weist eine Außenumhüllung 222 und eine Betätigungseinrichtung 226 auf.

Die Außenumhüllung 222 besteht aus einem ersten Element 222a und einem zweiten Element 222b. Die beiden Elemente 222a, 222b sind über zwei Gelenke 221, welche in radialer Richtung gesehen einander gegenüberliegend angeordnet sind, miteinander gelenkig verbunden. Die beiden Gelenke 221 befinden sich allerdings nicht an dem in Vorschubrichtung hinteren Ende der Außenumhüllung 222, sondern lediglich in einem in Vorschubrichtung gesehen hinteren Bereich der Außenumhüllung 222 ein wenig in Vorschubrichtung von dem hinteren Ende der Außenumhüllung 222 ausgehend gesehen nach vorne versetzt. Die Gelenke sind in der vorliegenden Ausführung als nicht-brechende Materialverbindungen ausgeführt.

In Vorschubrichtung gesehen hinter den Gelenken 221 sind bei beiden Elementen 222a, 222b Materialausnehmungen vorgesehen, welche eine Öffnungsbegrenzung 223 bilden. Diese Öffnungsbegrenzung 223 begrenzt die Relativbewegung der beiden Elemente 222a, 222b während der Überführung von der Abdeck-Position in die Freigabe-Position und verhindert somit ein Verklemmen der Elemente 222a, 222b mit dem chirurgischen Instrument 110, was bei einem möglicherweise gewünschten Herausziehen der Schutzabdeckung 220 hinderlich sein kann.

In der Abdeck-Position liegen die beiden Elemente 222a, 222b in einem Anlagebereich 224 lose aneinander an. Somit wird keine nennenswerte Kraft benötigt, um eine Relativbewegung der beiden Elemente 222a, 222b zueinander zu realisieren.

Das erste Element der 222a der Außenumhüllung 222 ist mit einem ersten Zugelement 226a der Betätigungseinrichtung 226 über einen ersten Vorsprungsabschnitt 225a verbunden und das zweite Element der 222b der Außenumhüllung 222 ist mit einem zweiten Zugelement 226b der Betätigungseinrichtung 226 über einen zweiten Vorsprungsabschnitt 225b verbunden.

Die Zugelemente 226a, 226b sind als bidirektional-kraftübertragende Kunststoffstäbe ausgebildet, die sich in ihrer Geometrie an der Geometrie des chirurgischen Instruments 110 orientieren, um somit einen möglichst ergonomischen Querschnitt der Operationseinheit 100 zu erzielen.

Die Vorsprungsabschnitte 225a, 225b sind als integrale Komponenten der Zugelemente 226a, 226b und/oder der beiden Elemente 222a, 222b der Außenumhüllung 222 ausgebildet. Die Vorsprungsabschnitte 225a, 225b verhindern ein ungewolltes Überführen der Außenumhüllung 222 von der Abdeck-Position in die Freigabe-Position, solange die Zugelemente 226a, 226b an ihrem der Außenumhüllung 222 abgewandten Ende arretiert und/oder fixiert sind.

Die beiden Zugelemente 226a, 226b weisen ein gemeinsames Halteelement 227 auf, welches, wie insbesondere aus Fig. 7 ersichtlich, die beiden Zugelemente 226a, 226b quer zur Vorschubrichtung miteinander verbindet und gleichzeitig die beiden Zugelemente 226a, 226b in im Wesentlichen Vorschubrichtung verschiebbar an dem chirurgischen Instrument 110 befestigt.

Die gesamte Schutzabdeckung 220 ist bevorzugt einteilig, insbesondere aus einen einteiligen Kunststoffbauteil, hergestellt. Dies gilt sowohl für die soeben vorgestellte erste Ausführungsform als auch für alle folgenden Ausführungsformen.

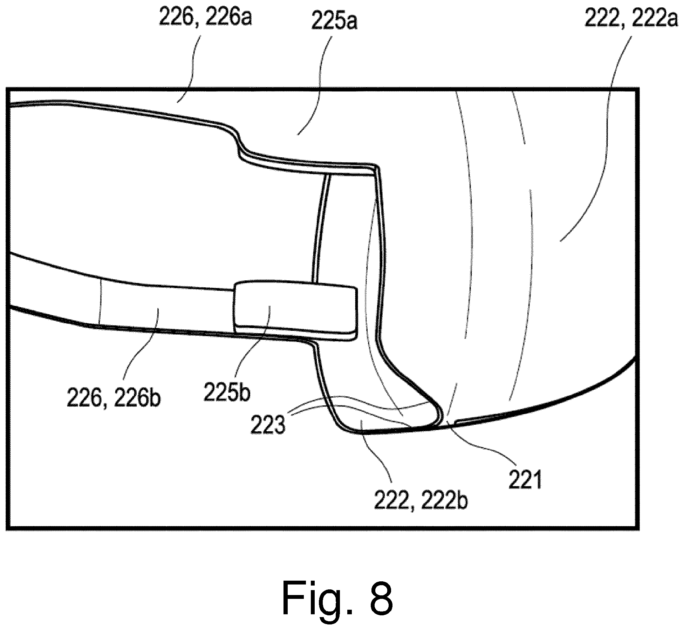

Fig. 8 zeigt eine Schutzabdeckung 220 nach einer zweiten Ausführungsform der vorliegenden Erfindung in einer Detailansicht. Diese zweite Ausführungsform ist im Wesentlichen identisch zu der vorstehend beschriebenen ersten Ausführungsform, sodass alle nicht im Folgenden als abweichend beschriebenen Merkmale als identisch angesehen werden können. Die Ausführungsform der Fig. 8 unterscheidet sich von der Ausführungsform der Fig. 6 und 7 dahingehend, dass die Vorsprungsabschnitte 225a, 225b eine abweichende Geometrie aufweisen. Die Vorsprungsabschnitte 225a, 225b sind insbesondere kürzer, und insbesondere auch dicker, als die der ersten Ausführungsform.

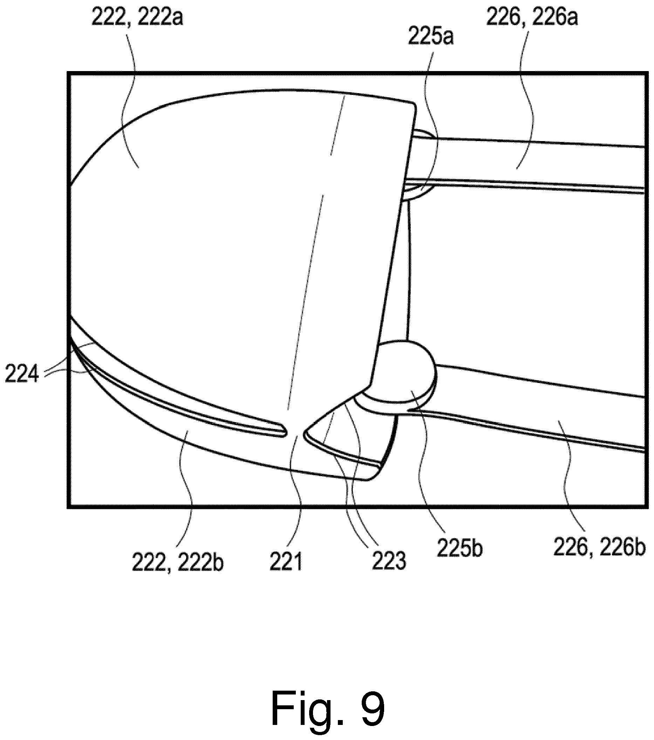

Fig. 9 zeigt eine Schutzabdeckung 220 nach einer dritten Ausführungsform der vorliegenden Erfindung in einer Detailansicht. Diese dritte Ausführungsform ist im Wesentlichen identisch zu der vorstehend beschriebenen ersten Ausführungsform, sodass alle nicht im Folgenden als abweichend beschriebenen Merkmale als identisch angesehen werden können. Die Ausführungsform der Fig. 9 unterscheidet sich von der Ausführungsform der Fig. 6 und 7 dahingehend, dass die Vorsprungsabschnitte 225a, 225b eine abweichende Geometrie aufweisen. Die Vorsprungsabschnitte 225a, 225b sind in diesem Fall tropfenförmig ausgebildet.

Die Geometrie der Vorsprungsabschnitte 225a, 225b erfüllt dabei die nachfolgende Aufgabe: Sperren einer Relativbewegung der Abschnitte 222a, 222b zueinander, sofern die Zugelemente 226a, 226b an ihrem der Außenumhüllung 222 abgewandten Ende arretiert und/oder fixiert sind.

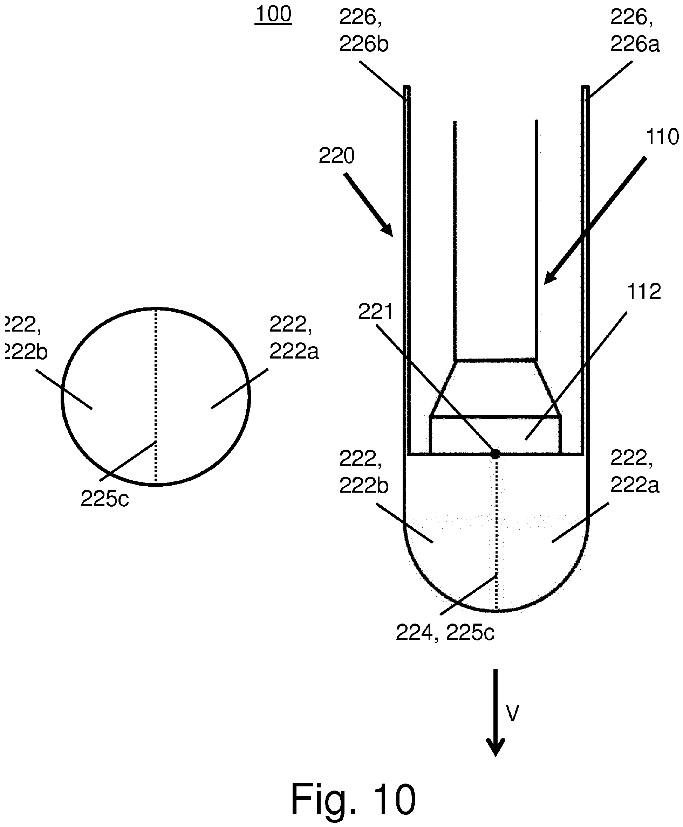

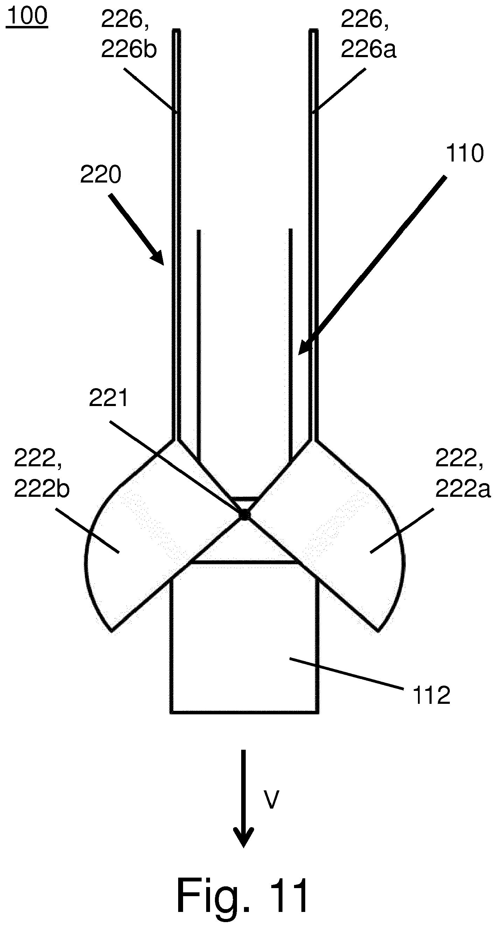

Fig. 10, 11 und 12 zeigen eine Schutzabdeckung 220 nach einer vierten Ausführungsform der vorliegenden Erfindung in der Abdeck-Position (Fig. 10) und in der Freigabe-Position (Fig. 11). Die Schutzabdeckung 220 weist eine Außenumhüllung 222 und eine Betätigungseinrichtung 226 auf.

Die Außenumhüllung 222 besteht aus einem ersten Element 222a und einem zweiten Element 222b. Die beiden Elemente 222a, 222b sind über zwei Gelenke 221, welche in radialer Richtung gesehen einander gegenüberliegend angeordnet sind, miteinander gelenkig verbunden. Die Gelenke 221 sind in der vorliegenden Ausführung als nicht-brechende Materialverbindungen ausgeführt.

Des Weiteren sind die beiden Elemente 222a, 222b über einen Materialfilm 225 in der Abdeck-Position (Fig. 10) in Form einer Materialausdünnung 225c miteinander verbunden. Die Materialausdünnung erstreckt sich über den gesamten Anlagebereich 224, welcher zwischen den beiden Gelenken 221 erstreckt, sodass in der Abdeck-Position eine in Vorschubrichtung von vorne gesehen geschlossene Oberfläche realisiert ist, wie sich dies auch aus der Vorderansicht in Fig. 10 (Fig. 10, links) ergibt. Die Materialausdünnung 225c ist durchgängig und insbesondere nicht perforiert.

Das erste Element 222a der Außenumhüllung 222 ist mit einem ersten Zugelement 226a der Betätigungseinrichtung 226 verbunden und das zweite Element 222b der Außenumhüllung 222 ist mit einem zweiten Zugelement 226b der Betätigungseinrichtung 226 verbunden.

Die Zugelemente 226a, 226b sind als bidirektional-kraftübertragende Kunststoffstäbe ausgebildet, die sich in ihrer Geometrie an der Geometrie des chirurgischen Instruments 110 orientieren, um somit einen möglichst ergonomischen Querschnitt der Operationseinheit 100 zu erzielen.

Die beiden Zugelemente 226a, 226b weisen ein gemeinsames Halteelement (nicht gezeigt) auf, welches die beiden Zugelemente 226a, 226b quer zur Vorschubrichtung miteinander verbindet und gleichzeitig die beiden Zugelemente 226a, 226b in im Wesentlichen Vorschubrichtung verschiebbar an dem chirurgischen Instrument 110 befestigt.

Fig. 11 zeigt die Schutzabdeckung der Fig. 10 in der Freigabe-Position. Während der Überführung der Schutzabdeckung 220 von der Abdeck-Position in die Freigabe-Position bricht die Außenumhüllung an der Materialausdünnung 225c auf einen Zug an den Zugelementen 226a, 226b hin auf und die beiden Elemente 222a, 222b der Außenumhüllung 222 bewegen sich um die durch die Gelenke 221 realisierte Schwenkachse voneinander weg und geben so den Kopfbereich 112 des chirurgischen Instruments 110 frei.

Die gesamte Schutzabdeckung 220 ist bevorzugt einteilig, insbesondere als ein einteiliges Kunststoffbauteil, hergestellt. Dies gilt sowohl für die soeben vorgestellte erste Ausführungsform als auch für die folgende Ausführungsform.

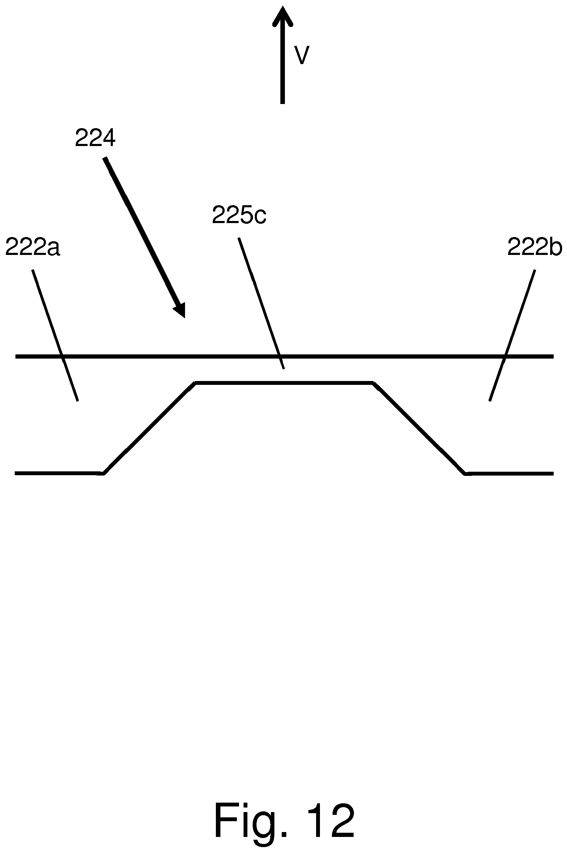

Fig. 12 zeigt den Anlagebereich 224 zwischen den beiden Elementen 222a, 222b der Außenumhüllung 222. Die Materialausdünnung 225c ist derart gestaltet, dass die Schutzabdeckung 220 aus der Vorschubrichtung V von vorne betrachtet eine durchgängige, glatte Oberfläche aufweist.

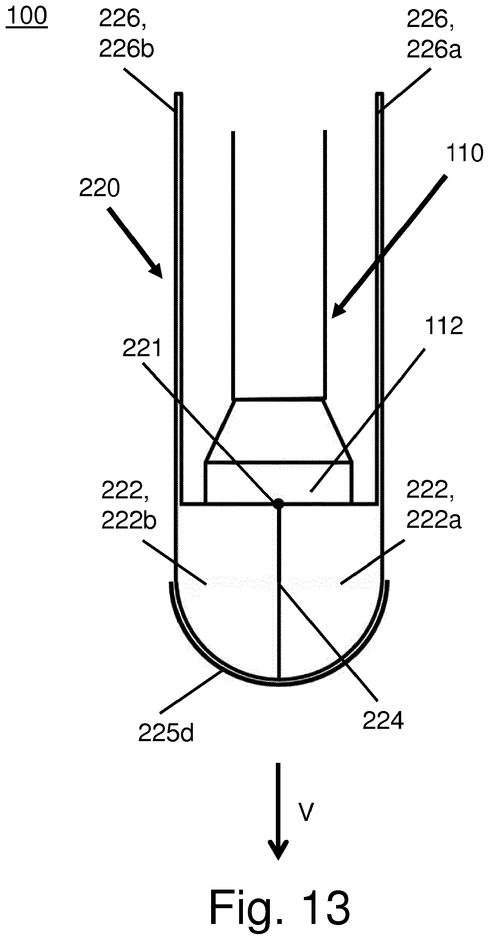

Fig. 13 zeigt eine Schutzabdeckung 220 nach der fünften Ausführungsform der vorliegenden Erfindung in einer Detailansicht. Diese fünfte Ausführungsform ist im Wesentlichen identisch zu der vorstehend beschriebenen Ausführungsform, sodass alle nicht im Folgenden als abweichend beschriebenen Merkmale als identisch angesehen werden können. Die Ausführungsform der Fig. 13 unterscheidet sich von der Ausführungsform der Fig. 10, 11 und 12 dahingehend, dass die beiden Elemente 222a, 222b der Außenumhüllung 222 nicht durch eine Materialausdünnung miteinander verbunden sind, sondern durch einen separat aufgebrachten Schutzfilm 225b. Die beiden Elemente 222a, 222b liegen im Anlagebereich 224 lose aneinander an und sind von dem Schutzfilm 225b bedeckt.

Der Schutzfilm 225d besteht in dieser Ausführung ebenfalls aus Kunststoff wie die Schutzabdeckung 220, aber nicht notwendigerweise aus demselben Kunststoff. Durch den Schutzfilm werden die Gleiteigenschaften der Schutzabdeckung innerhalb des Körpers weiter verbessert und können unabhängig von der Wahl des Materials der Schutzabdeckung 220 optimiert werden.

Während der Überführung der Schutzabdeckung 220 von der Abdeck-Position in die Freigabe-Position bricht der Schutzfilm 225d auf und die beiden Elemente 222a. 222b bewegen sich wie oben beschrieben um die Gelenke 221 herum voneinander weg, um den Kopfbereich 112 des chirurgischen Instruments 110 freizugeben bzw. freizulegen.

**Bezugszeichenliste**

| | |
|---|---|
| 100 | Operationseinheit |
| 110 | chirurgisches Instrument |
| 112 | Kopfbereich des chirurgischen Instruments 110 |
| 114 | Stift |
| 116 | Spitze des Stifts 114 |
| 120 | Schutzabdeckung |
| 122 | Außenumhüllung |
| 122a | erstes Element der Außenumhüllung 122 |
| 122b | zweites Element der Außenumhüllung 122 |
| 124 | Perforation |
| 126 | Zuganordnung |
| 128 | Sollbruchstelle |
| 220 | Schutzabdeckung |
| 221 | Gelenk |
| 222 | Außenumhüllung |
| 222a | erstes Segment der Außenumhüllung 222 |
| 222b | zweites Segment der Außenumhüllung 222 |
| 223 | Öffnungsbegrenzung |
| 224 | Anlagebereich |
| 225 | Materialfilm |
| 225a | erster Vorsprungsabschnitt |
| 225b | zweiter Vorsprungsabschnitt |
| 225c | Materialausdünnung |
| 225d | Schutzfilm |
| 226 | Betätigungseinrichtung |
| 226a | erstes Zugelement der Betätigungseinrichtung 226 |
| 226b | zweites Zugelement der Betätigungseinrichtung 226 |
| 227 | Halteelement |
| V | Vorschubrichtung |
| Z | Zugrichtung |

## Patentansprüche

1. Schutzabdeckung **(220)** für chirurgische Instrumente **(110),** insbesondere für Klammernahtgeräte, aufweisend:
eine Außenumhüllung **(222),** welche eingerichtet ist, wenigstens teilweise einen Kopfbereich **(112)** des chirurgischen Instruments **(110)** abzudecken;
eine Betätigungseinrichtung **(226),** welche mit der Außenumhüllung **(222)** wenigstens unidirektional kraftübertragend verbunden ist und aus wenigstens einem Zugelement **(226a, 226b)** gebildet ist,
wobei die Außenumhüllung **(222)** aus wenigstens einem ersten Segment **(222a)** und aus wenigstens einem zweiten Segment **(222b)** gebildet ist,
wobei das wenigstens eine erste Segment **(222a)** und das wenigstens eine zweite Segment **(222b)** mit Hilfe der Betätigungseinrichtung **(226)** von einer Abdeck-Position in eine Freigabe-Position überführbar sind,
wobei das wenigstens eine erste Segment (222a) und das wenigstens eine zweite Segment (222b) durch wenigstens ein Gelenk (221) miteinander verbunden sind,
**dadurch gekennzeichnet, dass**
die Betätigungseinrichtung **(226)** aus wenigstens einem ersten Zugelement **(226a)** und aus wenigstens einem zweiten Zugelement **(226b)** gebildet ist und
wobei das wenigstens eine erste Segment **(222a)** mit dem wenigstens einen ersten Zugelement **(226a)** über einen ersten Vorsprungsabschnitt **(225a)** verbunden ist und das wenigstens eine zweite Segment **(222b)** mit dem wenigstens einen zweiten Zugelement **(226b)** über einen zweiten Vorsprungsabschnitt **(225b)** verbunden ist.

2. Schutzabdeckung nach Anspruch 1, wobei das wenigstens eine erste Segment **(222a)** und das wenigstens eine zweite Segment **(222b)** in einem Anlagebereich **(224)** aneinander, insbesondere lose, anliegen.

3. Schutzabdeckung nach einem der vorangegangenen Ansprüche, wobei der wenigstens eine erste Vorsprungsabschnitt **(225a)** und der wenigstens eine zweite Vorsprungsabschnitt **(225b)** eingerichtet sind, das wenigstens eine erste Segment **(222a)** und das wenigstens eine zweite Segment **(222b)** in ihrer Position zu halten, bis eine Kraft über die Betätigungseinrichtung **(226)** an die Segmente **(222a, 222b)** übertragen wird,
und / oder
der wenigstens eine erste Vorsprungsabschnitt **(225a)** und/oder der wenigstens eine zweite Vorsprungsabschnitt **(225b)** durch eine Querschnittsverdickung der Betätigungseinrichtung **(226)** gebildet ist.

4. Schutzabdeckung nach einem der vorangegangenen Ansprüche, wobei das wenigstens eine erste Segment **(222a)** und das wenigstens eine zweite Segment **(222b)** in Vorschubrichtung gesehen hinter dem wenigstens einen Gelenk **(221)** jeweils eine Öffnungsbegrenzung **(223)** aufweisen, welche die Entfernung der in Vorschubrichtung gesehen vorderen Bereiche der Segmente **(222a, 222b)** zueinander in der Freigabeposition begrenzt.

5. Schutzabdeckung nach einem der vorangegangenen Ansprüche, wobei die Schutzabdeckung **(220)** im Wesentlichen aus Kunststoff besteht,
und / oder
wobei die Außenumhüllung **(222)** eingerichtet ist, das chirurgische Instrument **(110)** in seinem Kopfbereich **(112)** im Wesentlichen vollständig abzudecken.

6. Schutzabdeckung nach einem der vorangegangenen Ansprüche, wobei die Zugelemente **(226a, 226b)** ein Halteelement **(227)** aufweisen, welches eingerichtet ist, die Zugelemente **(226a, 226b)** an dem chirurgischen Instrument **(110)** zu fixieren.

7. Schutzabdeckung nach einem der vorangegangenen Ansprüche, wobei die Schutzabdeckung **(220)** in ihrer geometrischen Gestaltung eines in Vorschubrichtung gesehen vorderen Bereichs ein Kugelsegment aufweist.

8. Schutzabdeckung nach einem der vorangegangenen Ansprüche, wobei die Außenumhüllung **(222)** einen Materialfilm **(225)** aufweist, welcher in der Abdeckposition die beiden Segmente **(222a, 222b)** der Außenumhüllung **(222)** miteinander verbindet, und
der Materialfilm **(225)** als Materialausdünnung **(225c)** und/oder als Schutzfilm **(225d)** ausgebildet ist,
und / oder
wobei der Materialfilm **(225)** aus einem Kunststoffilm oder einem Papierfilm oder einem Metallfilm oder einem Schichtverbund der vorgenannten Filmarten gebildet ist.

9. Schutzabdeckung nach Anspruch 8, wobei der Materialfilm **(225)** als Schutzfilm **(225d)** ausgebildet ist und der Schutzfilm **(225d)** an der Außenumhüllung **(222)** befestigt ist; und/oder
der Schutzfilm **(225d)** auf die Außenumhüllung **(222)** aufgetragen ist; und/oder der Schutzfilm **(225d)** in der Vorschubrichtung von vorne gesehen in dem von der Außenumhüllung **(222)** wenigstens teilweise abgedeckten Kopfbereich **(112)** des chirurgischen Instruments **(110)** durchgängig ist; und/oder
der Schutzfilm **(225d)** keine Perforationen aufweist.

10. Schutzabdeckung nach Anspruch 8 in direkter oder indirekter Abhängigkeit von Anspruch 2, wobei der Materialfilm **(225)** als Schutzfilm **(225d)** ausgebildet ist und der Schutzfilm **(225d)** den Anlagebereich **(224)** des wenigstens einen ersten Abschnitts **(222a)** und des wenigstens einen zweiten Abschnitts **(222b)** in der Vorschubrichtung von vorne gesehen vollständig abdeckt.

11. Schutzabdeckung nach einem der vorangegangenen Ansprüche, wobei die Schutzabdeckung **(220)** zwei Gelenke **(221)** aufweist, welche in Vorschubrichtung gesehen in einem hinteren Bereich der Außenumhüllung **(222)** angeordnet sind.

12. Operationseinheit **(100),** aufweisend:
ein chirurgisches Instrument **(110),** insbesondere ein Klammernahtgerät; und
eine Schutzabdeckung **(220)** nach einem der Ansprüche 1 bis 11, wobei die Schutzabdeckung **(220)** an dem chirurgischen Instrument **(110)** angeordnet ist.

13. Operationseinheit nach Anspruch 12,
wobei die Schutzabdeckung **(220)** wenigstens im Wesentlichen aus Kunststoff besteht; und/oder
wobei die Schutzabdeckung **(220)** so gestaltet ist, dass sie zumindest einen Kopfbereich **(112)** des chirurgischen Instruments **(110)** wenigstens teilweise abdeckt.

14. Operationseinheit nach einem der Ansprüche 12 oder 13, wobei wenigstens zwei getrennt sterilisierte Verpackungseinheiten, nämlich wenigstens ein chirurgisches Instrument **(110),** insbesondere ein Klammernahtgerät, und wenigstens eine Schutzabdeckung **(220)** vorgesehen sind.

15. Operationseinheit nach einem der Ansprüche 12 bis 14, welche zur Verwendung bei wenigstens einer der folgenden Indikationen, insbesondere Klammernahtverbindungen, vorgesehen ist:
(a) Speiseröhre zu Magen und Magen zu Speiseröhre;
(b) Enddarm zu Dickdarm und Dickdarm zu Enddarm;
(c) Dickdarm zu Dickdarm;
(d) Dickdarm zu Dünndarm und Dünndarm zu Dickdarm;
(e) Dünndarm zu Dünndarm;
(f) Magen zu Dünndarm und Dünndarm zu Magen;
(g) Proktologische Eingriffe, insbesondere zur Schleimhautabtragung; und
(h) Vollwandresektionen.

## Claims

1. Protective cover **(220)** for surgical instruments **(110),** in particular for stapling suture devices, comprising:
an outer sheath **(222)** adapted to at least partially cover a head portion **(112) of** the surgical instrument **(110);**
an actuating device **(226),** which is at least unidirectionally force-transmittingly connected to the outer sheath **(222)** and is formed of at least one pulling element **(226a, 226b),**
wherein the outer sheath **(222)** is formed of at least one first segment **(222a)** and of at least one second segment **(222b),**
wherein the at least one first segment **(222a)** and the at least one second segment **(222b)** are transferable from a cover position to a release position by means of the actuating device **(226),**
wherein the at least one first segment **(222a)** and the at least one second segment **(222b)** are connected to each other by at least one joint **(221),**
**characterized in that**
the actuating device **(226)** is formed of at least one first pulling element **(226a)** and of at least one second pulling element **(226b),** and
wherein the at least one first segment **(222a)** is connected to the at least one first pulling element **(226a)** via a first projection portion **(225a)** and the at least one second segment **(222b)** is connected to the at least one second pulling element **(226b)** via a second projection portion **(225b).**

2. Protective cover according to claim 1, wherein the at least one first segment **(222a)** and the at least one second segment **(222b)** abut one another, in particular loosely, in an abutment area **(224).**

3. Protective cover according to any one of the preceding claims, wherein the at least one first projection portion **(225a)** and the at least one second projection portion **(225b)** are arranged to hold the at least one first segment **(222a)** and the at least one second segment **(222b)** in their position until a force is transmitted to the segments **(222a, 222b)** via the actuating device **(226),**
and / or
the at least one first projection portion **(225a)** and/or the at least one second projection portion **(225b)** is formed by a cross-sectional thickening of the actuating device **(226).**

4. Protective cover according to any one of the preceding claims, wherein the at least one first segment **(222a)** and the at least one second segment **(222b)** each have, behind the at least one joint **(221)** as viewed in the feed direction, an opening limitation **(223)** which limits the distance of the front regions, as viewed in the feed direction, of the segments **(222a, 222b)** from one another in the release position.

5. Protective cover according to any one of the preceding claims, wherein the protective cover **(220)** consists essentially of plastic,
and / or
wherein the outer sheath **(222)** is adapted to substantially completely cover the surgical instrument **(110)** in its head portion **(112).**

6. Protective cover according to any one of the preceding claims, wherein the pulling elements **(226a, 226b)** comprise a retaining member **(227)** configured to secure the pulling elements **(226a, 226b) to** the surgical instrument **(110).**

7. Protective cover according to any one of the preceding claims, wherein the protective cover **(220)** has a spherical segment in its geometric design of a front region as viewed in the feed direction.

8. Protective cover according to any one of the preceding claims, wherein the outer sheath **(222)** comprises a material film **(225)** which, in the cover position, connects the two segments **(222a, 222b) of** the outer sheath **(222)** to each other, and
the material film **(225)** is formed as a material thinning **(225c)** and/or as a protective film **(225d),**
and / or
wherein the material film **(225)** is formed of a plastic film or a paper film or a metal film or a layered composite of the aforementioned types of films.

9. Protective cover according to claim 8, wherein the material film **(225)** is formed as a protective film **(225d)** and the protective film **(225d)** is attached to the outer sheath **(222);** and/or
the protective film **(225d)** is applied to the outer sheath **(222);** and/or
the protective film **(225d)** is continuous in the head portion **(112)** of the surgical instrument (**110),** which is at least partially covered by the outer sheath **(222),** as viewed from the front in the feed direction; and/or
the protective film **(225d)** has no perforations.

10. Protective cover according to claim 8 in direct or indirect dependence on claim 2, wherein the material film **(225)** is formed as a protective film **(225d)** and the protective film **(225d)** completely covers the abutment area **(224)** of the at least one first portion **(222a)** and of the at least one second portion **(222b)** as viewed from the front in the feed direction.

11. Protective cover according to any one of the preceding claims, wherein the protective cover **(220)** comprises two joints **(221),** which are arranged in a rear portion of the outer sheath **(222)** as viewed in the feed direction.

12. Surgical unit **(100),** comprising:
a surgical instrument **(110),** in particular a stapling suture device; and
a protective cover **(220)** according to any one of claims 1 to 11, wherein the protective cover **(220)** is arranged at the surgical instrument **(110).**

13. Surgical unit according to claim 12,
wherein the protective cover **(220)** is at least substantially made of plastic; and/or
wherein the protective cover **(220)** is designed to at least partially cover at least a head portion **(112)** of the surgical instrument **(110).**

14. Surgical unit according to one of claims 12 or 13, wherein at least two separately sterilized packaging units, namely at least one surgical instrument **(110),** in particular a stapling suture device, and at least one protective cover **(220)** are provided.

15. Surgical unit according to any one of claims 12 to 14, which is intended for use in at least one of the following indications, in particular stapling suture connections:
(a) Esophagus to stomach and stomach to esophagus;
(b) rectum to colon and colon to rectum;
(c) Colon to colon;
(d) Colon to small intestine and small intestine to colon;
(e) Small intestine to small intestine;
(f) Stomach to small intestine and small intestine to stomach;
(g) Proctologic surgeries, particularly for mucosal ablation;
and
(h) Full thickness resections.

## Revendications

1. Couvercle de protection (220) pour des instruments chirurgicaux (110), en particulier pour des appareils de suture par agrafage, présentant :
une enveloppe extérieure (222), laquelle est conçue pour recouvrir au moins partiellement une zone de tête (112) de l'instrument chirurgical (110) ;
un équipement d'actionnement (226), lequel est relié à l'enveloppe extérieure (222) de façon à transmettre une force au moins unidirectionnellement et est constitué d'au moins un élément de traction (226a, 226b),
dans lequel l'enveloppe extérieure (222) est constituée d'au moins un premier segment (222a) et d'au moins un deuxième segment (222b),
dans lequel l'au moins un premier segment (222a) et l'au moins un deuxième segment (222b) peuvent être passés d'une position de recouvrement à une position de libération à l'aide de l'équipement d'actionnement (226),
dans lequel l'au moins un premier segment (222a) et l'au moins un deuxième segment (222b) sont reliés l'un à l'autre par au moins une articulation (221),
**caractérisé en ce que**
l'équipement d'actionnement (226) est constitué d'au moins un premier élément de traction (226a) et d'au moins un deuxième élément de traction (226b) et
dans lequel l'au moins un premier segment (222a) est relié à l'au moins un premier élément de traction (226a) via une première section faisant saillie (225a)
et l'au moins un deuxième segment (222b) est relié à l'au moins un deuxième élément de traction (226b) via une deuxième section faisant saillie (225b).

2. Couvercle de protection selon la revendication 1, dans lequel l'au moins un premier segment (222a) et l'au moins un deuxième segment (222b) s'appliquent l'un contre l'autre, en particulier de manière lâche, dans une zone d'application (224).

3. Couvercle de protection selon l'une des revendications précédentes, dans lequel l'au moins une première section faisant saillie (225a) et l'au moins une deuxième section faisant saillie (225b) sont conçues pour maintenir l'au moins un premier segment (222a) et l'au moins un deuxième segment (222b) dans leur position jusqu'à ce qu'une force soit transmise aux segments (222a, 222b) via l'équipement d'actionnement (226),
et/ou
l'au moins une première section faisant saillie (225a) et/ou l'au moins une deuxième section faisant saillie (225b) est constituée par un épaississement de section transversale de l'équipement d'actionnement (226).

4. Couvercle de protection selon l'une des revendications précédentes, dans lequel l'au moins un premier segment (222a) et l'au moins un deuxième segment (222b) présentent respectivement, vu dans la direction d'avancement derrière l'au moins une articulation (221), une limitation d'ouverture (223), laquelle limite dans la position de libération la distance entre les zones avant des segments (222a, 222b) vu dans la direction d'avancement.

5. Couvercle de protection selon l'une des revendications précédentes, dans lequel le couvercle de protection (220) se compose essentiellement de matière plastique,
et/ou
dans lequel l'enveloppe extérieure (222) est conçue pour recouvrir pour l'essentiel complètement l'instrument chirurgical (110) dans sa zone de tête (112).

6. Couvercle de protection selon l'une des revendications précédentes, dans lequel les éléments de traction (226a, 226b) présentent un élément de retenue (227), lequel est conçu pour fixer les éléments de traction (226a, 226b) contre l'instrument chirurgical (110).

7. Couvercle de protection selon l'une des revendications précédentes, dans lequel le couvercle de protection (220) présente un segment sphérique dans sa configuration géométrique d'une zone avant, vu dans le sens de l'avancement.

8. Couvercle de protection selon l'une des revendications précédentes, dans lequel l'enveloppe extérieure (222) présente un film de matériau (225), lequel relie l'un à l'autre les deux segments (222a, 222b) de l'enveloppe extérieure (222) dans la position de recouvrement, et
le film de matériau (225) est conçu en tant qu'amincissement de matériau (225c) et/ou en tant que film de protection (225d),
et/ou
dans lequel le film de matériau (225) est constitué d'un film de matière plastique ou d'un film de papier ou d'un film métallique ou d'un composite feuilleté des types de film susmentionnés.

9. Couvercle de protection selon la revendication 8, dans lequel le film de matériau (225) est conçu en tant que film de protection (225d) et le film de protection (225d) est fixé contre l'enveloppe extérieure (222) ; et/ou
le film de protection (225d) est apporté sur l'enveloppe extérieure (222) ; et/ou
le film de protection (225d), vu depuis l'avant dans la direction d'avancement, est continu dans la zone de tête (112) de l'instrument chirurgical (110) recouverte au moins partiellement par l'enveloppe extérieure (222) ; et/ou
le film de protection (225d) ne présente aucune perforation.

10. Couvercle de protection selon la revendication 8, en dépendance directe ou indirecte de la revendication 2, dans lequel le film de matériau (225) est conçu en tant que film de protection (225d) et le film de protection (225d), vu depuis l'avant dans la direction d'avancement, recouvre complètement la zone d'application (224) de l'au moins une première section (222a) et de l'au moins une deuxième section (222b).

11. Couvercle de protection selon l'une des revendications précédentes, dans lequel le couvercle de protection (220) présente deux articulations (221), lesquelles, vu dans la direction d'avancement, sont agencées dans une zone arrière de l'enveloppe extérieure (222).

12. Unité d'opération (100), présentant :
un instrument chirurgical (110), en particulier un appareil de suture par agrafage ; et
un couvercle de protection (220) selon l'une des revendications 1 à 11, dans laquelle le couvercle de protection (220) est agencé contre l'instrument chirurgical (110).

13. Unité d'opération selon la revendication 12,
dans laquelle le couvercle de protection (220) se compose au moins essentiellement de matière plastique ; et/ou
dans laquelle le couvercle de protection (220) est configuré de sorte qu'il recouvre au moins partiellement au moins une zone de tête (112) de l'instrument chirurgical (110).

14. Unité d'opération selon l'une des revendications 12 ou 13, dans laquelle sont prévues au moins deux unités d'emballage stérilisées séparément, à savoir au moins un instrument chirurgical (110), en particulier un appareil de suture par agrafage, et au moins un couvercle de protection (220).

15. Unité d'opération selon l'une des revendications 12 à 14, laquelle est prévue pour une utilisation dans le cas d'au moins l'une des indications suivantes, en particulier des assemblages par suture par agrafage :
a) de l'œsophage à l'estomac et de l'estomac à l'œsophage ;
b) du colon au gros intestin et du gros intestin au colon ;
c) du gros intestin au gros intestin ;
d) du gros intestin à l'intestin grêle et de l'intestin grêle au gros intestin ;
e) de l'intestin grêle à l'intestin grêle ;
f) de l'estomac à l'intestin grêle et de l'intestin grêle à l'estomac ;
g) interventions proctologiques, en particulier pour l'ablation de la muqueuse ; et
h) résections de la paroi complète.
